# EUROPEAN PATENT APPLICATION

(11) **EP 3 118 196 A1**
(43) Date of publication of application: **18.01.2017**
(21) Application number: 15760831.6
(22) Date of filing: 11.03.2015
(51) Int. Cl.: C07D 333/40, C07D 333/38

(54) **METHOD FOR PRODUCING HETEROARYLCARBOXYLIC ACID ESTER DERIVATIVE, AND PRODUCTION INTERMEDIATE OF SAME**

(30) Priority: 11.03.2014 JP 2014048091
(71) Applicant: EA Pharma Co., Ltd., Tokyo 104-0042 (JP)
(72) Inventor: TAKASHITA, Ryuta, Kawasaki-shi, Kanagawa 210-8681 (JP)
(74) Representative: Nicholls, Kathryn Margaret
(86) International application number: PCT/JP2015/057178
(87) International publication number: WO 2015/137408

(57) **Abstract**

Provided are a production method of compound (9) useful as a therapeutic drug for diabetes and a production intermediate therefor. (a) Diester (3) is synthesized by the reaction of compound (1) and compound (2), (b) ester (4) is synthesized by deprotection of diester (3) (or ester (4) is synthesized by the reaction of compound (5) and compound (2)), (c) ester (4) is converted to acid halide, (d) the acid halide is reacted with amidinophenol derivative, and (e) the obtained diester derivative is deprotected under acidic conditions, and converted to compound (9): 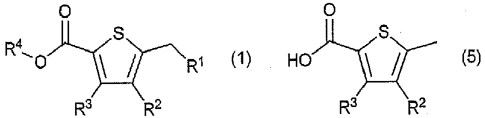 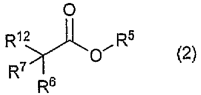 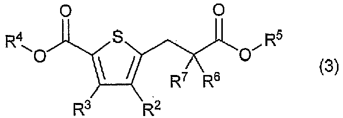 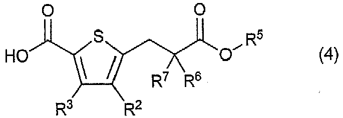 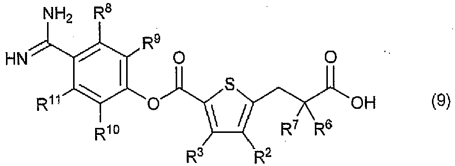 wherein R¹ is a halogen atom, R⁴ is a lower alkyl group, R⁵ is a lower alkyl group, and R¹² is a hydrogen atom or a halogen atom.

## Description

### Technical Field

The present invention relates to a novel production method of heteroarylcarboxylic acid ester derivatives, and production intermediates therefor. More particularly, the present invention relates to an efficient production method of a heteroarylcarboxylic acid ester derivative as a therapeutic drug for diabetes and a production intermediate therefor, and a production intermediate useful for such production method.

### Background Art

At present, insulin secretagogue (sulfonylurea), glucose absorption inhibitor (α-glucosidase inhibitor), insulin sensitizer (biguanide, thiazolidine derivative) and the like are clinically used as therapeutic drugs for diabetes. However, all of them still have problems in that they are accompanied by side effects such as hypoglycemia, diarrhea, lactic acidosis, edema and the like, show insufficient effect, and the like. As a new treatment or prophylactic drug for diabetes that satisfies clinical needs, patent document 1 and patent document 2 disclose heteroarylcarboxylic acid ester derivatives encompassed by the following formula (I), and it has been reported that representative compounds thereof show a superior blood glucose elevation suppressing effect in diabetes animal models.

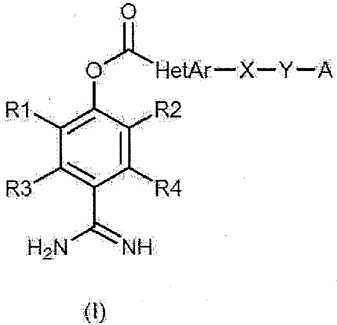

In patent document 1, a method shown by the following scheme is disclosed as a general synthesis method of a heteroarylcarboxylic acid ester derivative encompassed by the formula (I) (refer to patent document 1 for symbols in the formula).

A heteroarylcarboxylic acid ester derivative (F) of the formula (I) wherein X is a lower alkylene group or a lower alkenylene group, A is -OR5, and R5 is a lower alkyl group can be produced as follows.

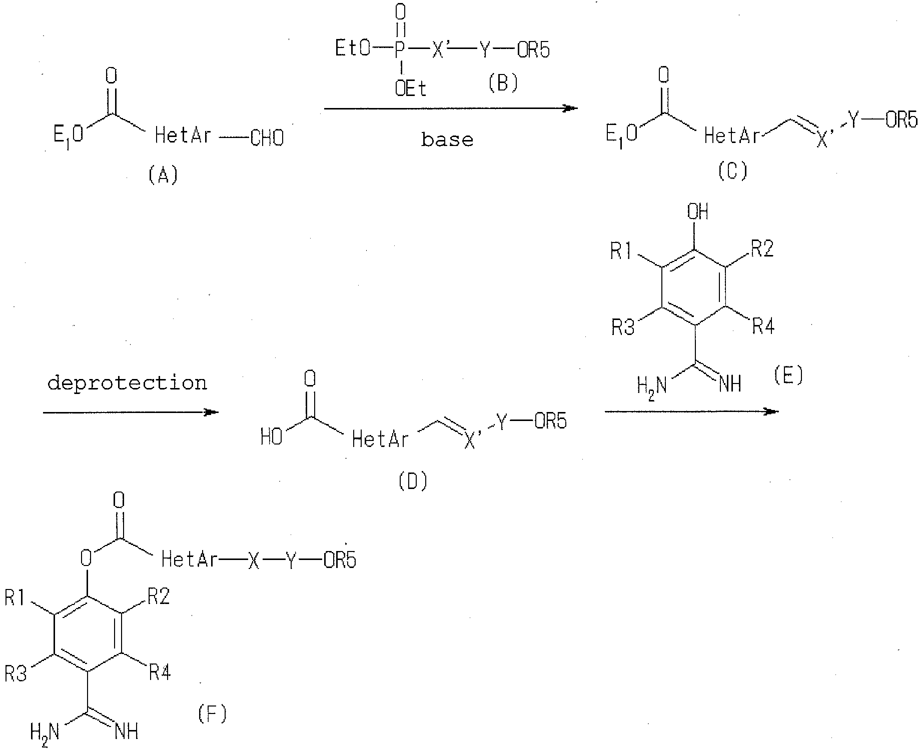

An object heteroarylcarboxylic acid ester derivative (F) wherein X is a lower alkenylene group can be produced by esterification of carboxylic acid derivative (D) and amidinophenol derivative (E), and a heteroarylcarboxylic acid ester derivative (F) wherein X is a lower alkylene group can be produced by a step of treating with a catalyst such as 10% palladium/carbon, in a solvent that does not adversely influence this reaction, such as methanol, ethanol, ethyl acetate and the like, under a hydrogen atmosphere at some stage of the production step.

A known method can be applied to the esterification reaction and, for example, (1) a method using an acid halide, (2) a method using a condensing agent and the like can be mentioned.

The (1) method using an acid halide is performed by reacting a carboxylic acid with thionyl chloride, oxalyl chloride and the like in a solvent that does not adversely influence this reaction such as dichloromethane and the like or without solvent in the presence or absence of a catalyst such as N,N-dimethylformamide and the like to give an acid chloride, and reacting the obtained acid chloride with an alcohol in a solvent that does not adversely influence this reaction such as dichloromethane, tetrahydrofuran and the like, in the presence of a base such as pyridine and triethylamine.

The (2) method using a condensing agent is performed by reacting a carboxylic acid and an alcohol in a solvent that does not adversely influence this reaction such as tetrahydrofuran, N,N-dimethylformamide, dichloromethane and the like, in the presence or absence of a base such as pyridine, triethylamine and the like by using a condensing agent such as 1-ethyl-3-(3'-dimethylaminopropyl)carbodiimide (WSC) or 1,3-dicyclohexylcarbodiimide and the like.

A heteroarylcarboxylic acid ester derivative (i) of the formula (I) wherein A is -OR5, and R5 is a hydrogen atom can be produced by subjecting ester derivative (H) obtained by using Wittig reagent (G) (wherein E₂ is a protecting group such as methyl group, ethyl group, isopropyl group, tert-butyl group, benzyl group and the like) instead of Wittig reagent (B) to, for example, hydrolysis by a base such as sodium hydroxide and the like, hydrolysis by an acid such as hydrochloric acid, trifluoroacetic acid and the like or deprotection by a treatment with 10% palladium/carbon etc. under a hydrogen atmosphere, and the like.

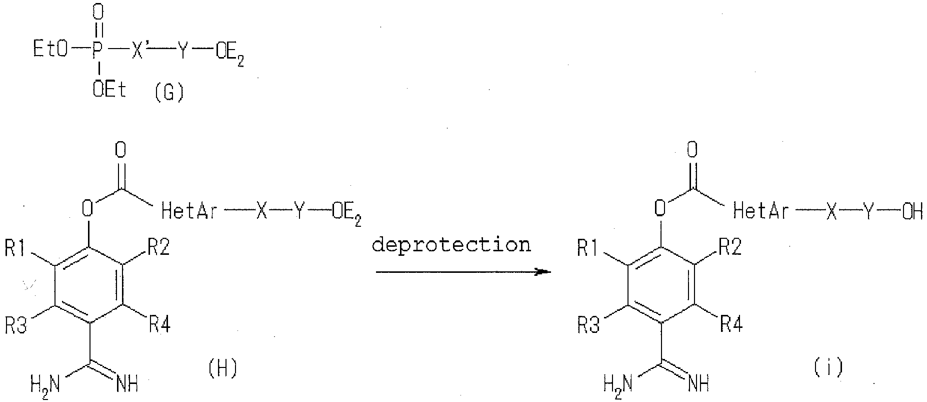

Also, patent document 2 provides a similar description of a general synthesis method of a heteroarylcarboxylic acid ester derivative encompassed by the formula (I).

However, in the Examples of patent document 1 and patent document 2, column chromatography is used for an isolation operation of ester derivative (F) and (i), and the yield thereof is not appropriate for an industrial process. Thus, while a heteroarylcarboxylic acid ester derivative encompassed by the formula (I) is expected as a useful therapeutic drug for diabetes, economic efficiency and productivity are poor by a conventional production method, and a new method capable of industrial production with good efficiency is desired.

### Document List

### Patent Documents

patent document 1: WO 2011/071048
patent document 2: WO 2013/187533

### Summary of the Invention

### Problems to be Solved by the Invention

An industrial production method for producing a heteroarylcarboxylic acid ester derivative encompassed by the formula (I) in a better yield with better quality than conventional methods is desired.

### Means of Solving the Problems

To solve the above-mentioned problem, intensive studies have been conducted and an industrial production method for producing heteroarylcarboxylic acid ester derivatives shown below and novel intermediates used therefor have been found, which resulted in the completion of the present invention.

Therefore, the present invention provides a production method of a heteroarylcarboxylic acid ester derivative represented by the following formula (9) suitable for industrialization and intermediates useful for the production thereof.

The present invention relates to the following.
[1] A production method of an ester derivative represented by the formula (4), or a chemically acceptable salt thereof: 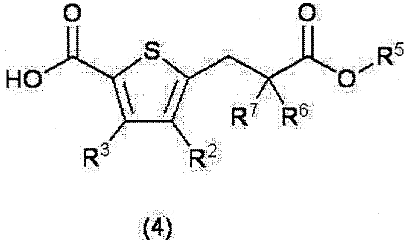
   wherein R² and R³ are the same or different and each is independently a hydrogen atom or a lower alkyl group,
   R⁵ is a tert-butyl group,
   R⁶ and R⁷ are the same or different and each is independently a hydrogen atom or a lower alkyl group optionally having a substituent (s), or R⁶ and R⁷ form a C₃₋₈ cycloalkane ring together with a carbon atom bonded thereto,
   the method comprising the following steps (g) and (h):
      (g) reacting a compound represented by the formula (1) and a compound represented by the formula (2) in the presence of (A) an alkyllithium or (B) a lithium complex prepared from an organic amine and an alkyllithium to give a compound represented by the formula (3): 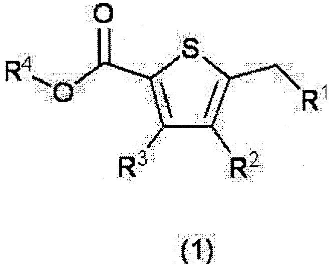 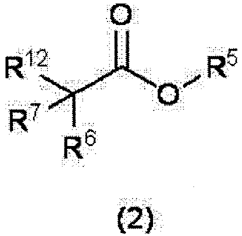 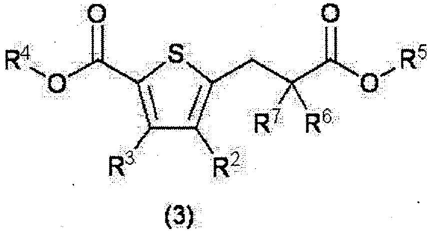
         wherein R¹ is a halogen atom,
         R⁴ is a methyl group, an ethyl group, an isopropyl group or an n-butyl group,
         R¹² is a hydrogen atom or a halogen atom, and
         other symbols are as defined above, and
      (h) hydrolyzing the compound represented by the formula (3) with a metal hydroxide in a solvent containing water and an alcohol to give an ester derivative represented by the formula (4), or a chemically acceptable salt thereof.
[2] The production method of the aforementioned [1], wherein the alkyllithium is n-butyllithium.
[3] A production method of an ester derivative represented by the formula (11), or a chemically acceptable salt thereof: 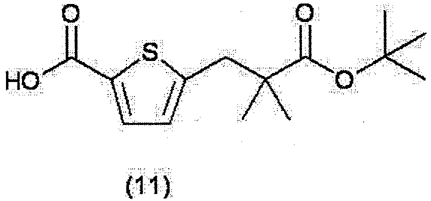 the method comprising the following steps (k) and (1):
   (k) reacting a compound represented by the formula (12) with a compound represented by the formula (13) in the presence of (A) n-butyllithium or (B) a lithium complex prepared from an organic amine selected from diisopropylamine and diisopropylethylamine, and n-butyllithium to give a compound represented by the formula (10): 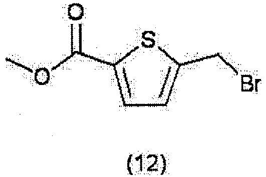 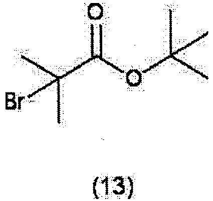 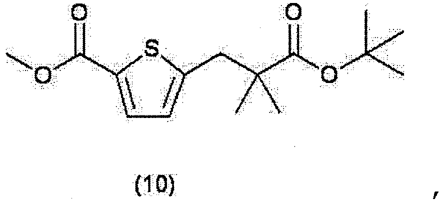 and
   (1) hydrolyzing the compound represented by the formula (10) with a metal hydroxide selected from sodium hydroxide, lithium hydroxide and potassium hydroxide in a solvent containing water and an alcohol to give an ester derivative represented by the formula (11), or a chemically acceptable salt thereof.
[4] A production method of an ester derivative represented by the formula (4), or a chemically acceptable salt thereof: 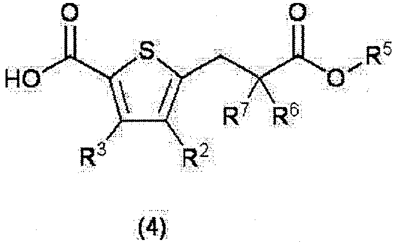
   wherein R² and R³ are the same or different and each is independently a hydrogen atom or a lower alkyl group optionally having a substituent(s),
   R⁵ is a lower alkyl group, and
   R⁶ and R⁷ are the same or different and each is independently a hydrogen atom or a lower alkyl group optionally having a substituent(s), or R⁶ and R⁷ form a C₃₋₈ cycloalkane ring together with a carbon atom bonded thereto,
   the method comprising step (m):
      (m) reacting a compound represented by the formula (5) with a compound represented by the formula (2) using a base to give an ester derivative represented by the formula (4), or a chemically acceptable salt thereof: 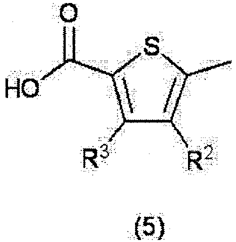 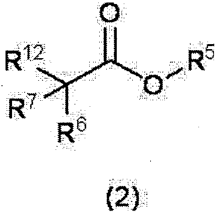
         wherein R¹² is a halogen atom, and
         other symbols are as defined above.
[5] A production method of an ester derivative represented by the formula (11), or a chemically acceptable salt thereof: 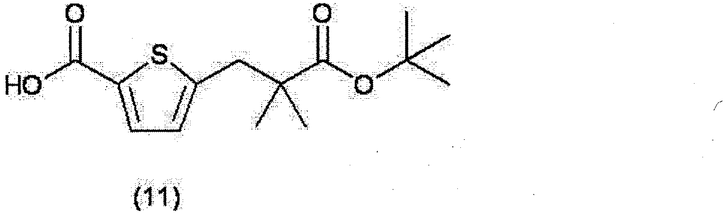 the method comprising step (n):
   (n) reacting a compound represented by the formula (14) with a compound represented by the formula (13) using sodium hydride and lithium diisopropylamide to give an ester derivative represented by the formula (11), or a chemically acceptable salt thereof: 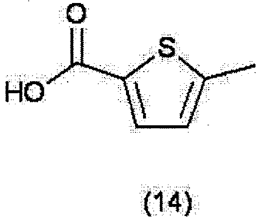 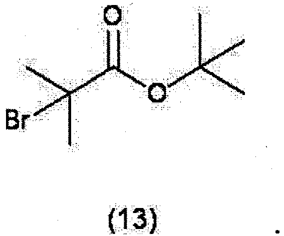
[6] A production method of a heteroarylcarboxylic acid ester derivative represented by the formula (9), or a chemically acceptable salt thereof: 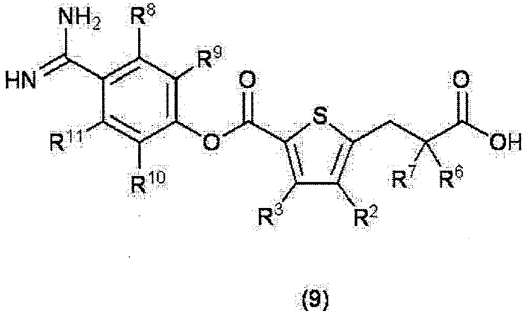
   wherein R² and R³ are the same or different and each is independently a hydrogen atom or a lower alkyl group optionally having a substituent(s),
   R⁶ and R⁷ are the same or different and each is independently a hydrogen atom or a lower alkyl group optionally having a substituent(s), or R⁶ and R⁷ form a C₃₋₈ cycloalkane ring together with a carbon atom bonded thereto, and
   R⁸, R⁹, R¹⁰ and R¹¹ are the same or different and each is independently a hydrogen atom or a halogen atom,
   the method comprising the following steps (o) to (q):
      (o) reacting the compound represented by the formula (4) with a halogenating agent to give an acid halide represented by the formula (6): 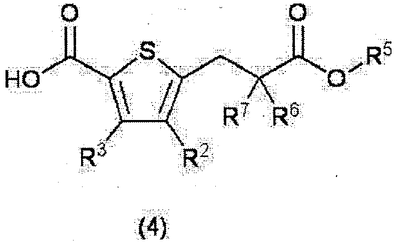 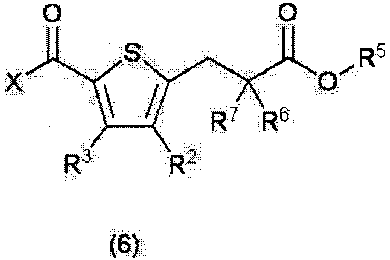
         wherein R⁵ is a lower alkyl group,
         X is a halogen atom, and
         other symbols are as defined above,
      (p) reacting the acid halide represented by the formula (6) with a compound represented by the formula (7) to give a compound represented by the formula (8), or a chemically acceptable salt thereof: 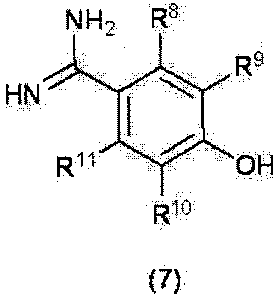 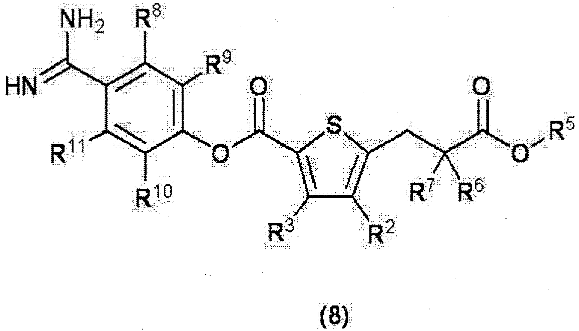 wherein the symbols are as defined above, and
      (q) deprotecting the compound represented by the formula (8), or a chemically acceptable salt thereof under acidic conditions to give a heteroarylcarboxylic acid ester derivative represented by the formula (9), or a chemically acceptable salt thereof.
[7] A production method of a heteroarylcarboxylic acid ester derivative represented by the formula (9), or a chemically acceptable salt thereof: 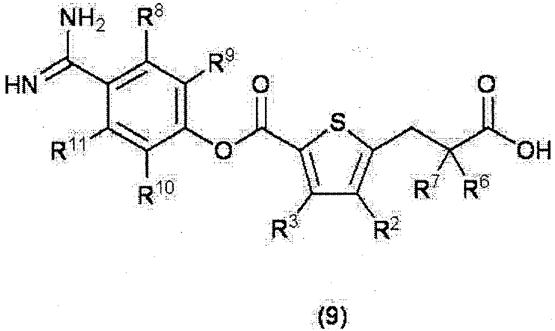
   wherein R² and R³ are the same or different and each is independently a hydrogen atom or a lower alkyl group optionally having a substituent(s),
   R⁶ and R⁷ are the same or different and each is independently a hydrogen atom or a lower alkyl group optionally having a substituent(s), or R⁶ and R⁷ form a C₃₋₈ cycloalkane ring together with a carbon atom bonded thereto, and
   R⁸, R⁹, R¹⁰ and R¹¹ are the same or different and each is independently a hydrogen atom or a halogen atom,
   the method comprising the following steps (r) to (t):
      (r) reacting a compound represented by the formula (4) with thionyl chloride or oxalyl chloride to give an acid chloride represented by the formula (16): 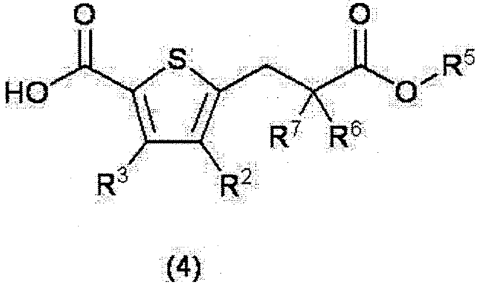 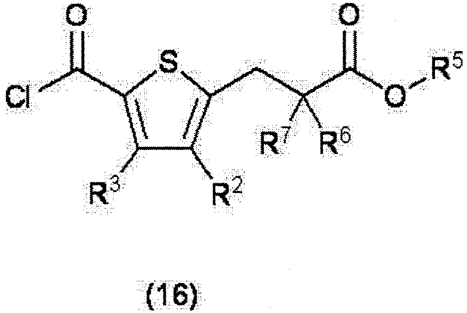
         wherein R⁵ is a lower alkyl group, and
         other symbols are as defined above,
      (s) reacting the acid chloride represented by the formula (16) with a compound represented by the formula (7) in the presence of an organic base to give a compound represented by the formula (8), or a chemically acceptable salt thereof: 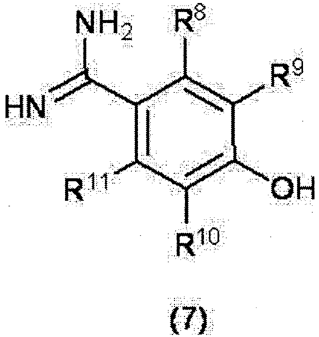 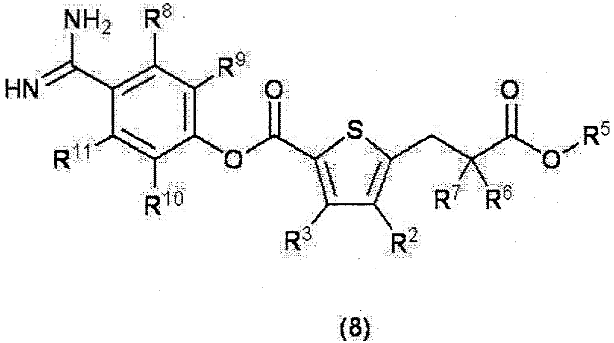 wherein each symbol is as defined above, and
      (t) deprotecting the compound represented by the formula (8), or a chemically acceptable salt thereof with HCl, HBr, sulfuric acid, methanesulfonic acid or trifluoroacetic acid to give a heteroarylcarboxylic acid ester derivative represented by the formula (9), or a chemically acceptable salt thereof.
[8] A production method of a heteroarylcarboxylic acid ester derivative represented by the formula (17), or a chemically acceptable salt thereof: 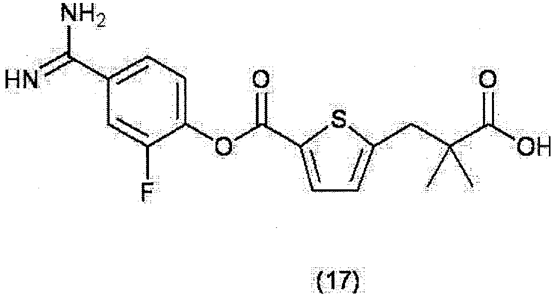 the method comprising the following steps (u) to (w):
   (u) reacting a compound represented by the formula (11) with thionyl chloride to give an acid chloride represented by the formula (18): 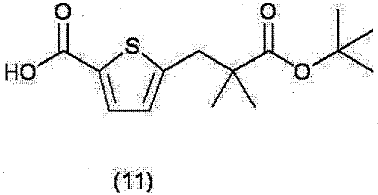 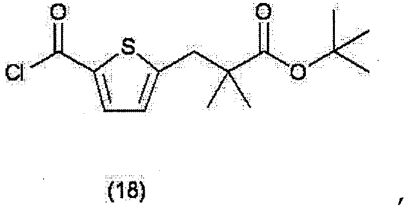
   (v) reacting the acid chloride represented by the formula (18) with a compound represented by the formula (19) in the presence of pyridine to give a compound represented by the formula (15), or a chemically acceptable salt thereof: 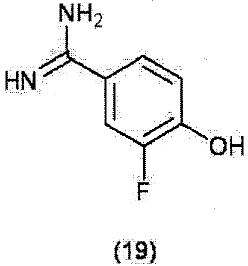 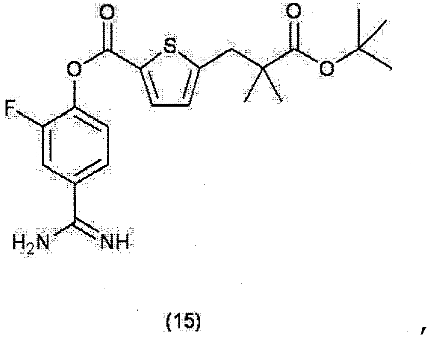 and
   (w) deprotecting a compound represented by the formula (15), or a chemically acceptable salt thereof in the presence of HCl to give a heteroarylcarboxylic acid ester derivative represented by the formula (17), or a chemically acceptable salt thereof.
[9] A compound represented by the following formula (20), or a chemically acceptable salt thereof: 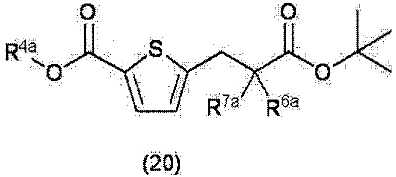
   wherein R^{4a} is a methyl group, an ethyl group, an isopropyl group or an n-butyl group,
   R^{6a} and R^{7a} are the same and are methyl groups or ethyl groups, or R^{6a} and R^{7a} form a cyclopropane ring, a cyclobutane ring or a cyclopentane ring together with a carbon atom bonded thereto.
[10] The compound of the aforementioned [9], wherein R^{6a} and R^{7a} are methyl groups, or a chemically acceptable salt thereof.
[11] The compound of the aforementioned [9], wherein R^{4a} is a methyl group, or a chemically acceptable salt thereof.
[12] A compound represented by the following formula (10), or a chemically acceptable salt thereof: 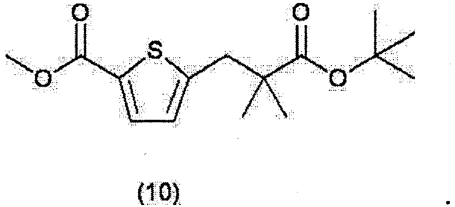
[13] A compound represented by the following formula (21), or a chemically acceptable salt thereof: 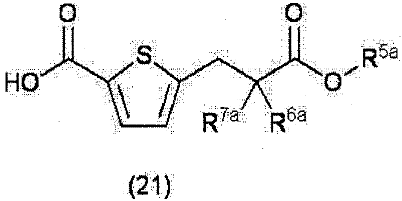
   wherein R^{5a} is a methyl group, an ethyl group, an isopropyl group, a tert-butyl group or an n-butyl group,
   R^{6a} and R^{7a} are the same and are methyl groups or ethyl groups, or R^{6a} and R^{7a} form a cyclopropane ring, a cyclobutane ring or a cyclopentane ring together with a carbon atom bonded thereto.
[14] The compound of the aforementioned [13], wherein R^{6a} and R^{7a} are methyl groups, or a chemically acceptable salt thereof.
[15] The compound of the aforementioned [13], wherein R^{5a} is a tert-butyl group, or a chemically acceptable salt thereof.
[16] A compound represented by the following formula (11), or a chemically acceptable salt thereof: 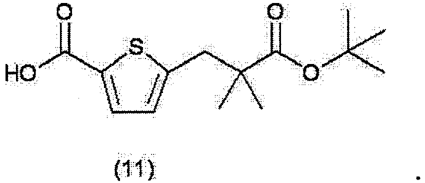
[17] A compound represented by the following formula (8), or a chemically acceptable salt thereof 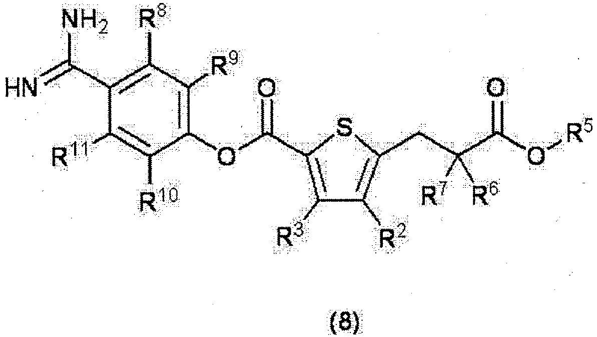
   wherein R² and R³ are the same or different and each is independently a hydrogen atom or a lower alkyl group optionally having a substituent(s),
   R⁵ is a lower alkyl group,
   R⁶ and R⁷ are the same or different and each is independently a hydrogen atom or a lower alkyl group optionally having a substituent(s), or R⁶ and R⁷ form a C₃₋₈ cycloalkane ring together with a carbon atom bonded thereto, and
   R⁸, R⁹, R¹⁰ and R¹¹ are the same or different and each is independently a hydrogen atom or a halogen atom.
[18] The compound of the aforementioned [17], wherein, in the formula (8), R² and R³ are hydrogen atoms, and
   R⁶ and R⁷ are the same or different and each is independently a methyl group, an ethyl group or a propyl group, or R⁶ and R⁷ form a cyclopropane ring, a cyclobutane ring or a cyclopentane ring together with a carbon atom bonded thereto, or a chemically acceptable salt thereof.
[19] A compound represented by the following formula (15), or a chemically acceptable salt thereof:

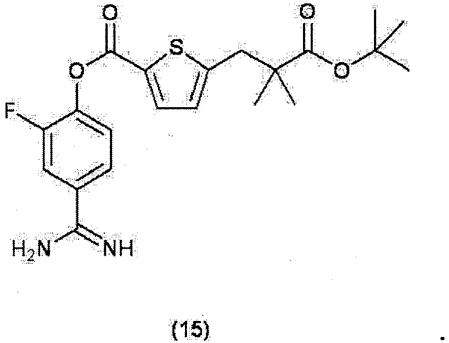

### Effect of the Invention

The present invention provides a production method suitable for the mass synthesis of heteroarylcarboxylic acid ester derivatives and their novel synthetic intermediates. In the production method of the present invention, since an ester derivative protected by a lower alkyl group is used as an intermediate, the resultant product can be crystallized and isolated conveniently by filtration. Using the production method of the present invention, the object compound, a heteroarylcarboxylic acid ester derivative, can be produced in a high yield and with high purity.

### Description of Embodiments

In the present specification, the term "optionally having a substituent(s)" means "being substituted or unsubstituted". Unless otherwise specified, the position and number of the substituents may be any, and are not particularly limited. The number of the substituents is preferably 1 to 5, more preferably 1 to 3. When substituted by two or more substituents, the substituents may be the same or different. Examples of the substituent include halogen atom, cyano group, phenyl group, lower alkyl group, lower acyl group, lower alkoxyl group, lower alkylthio group, and the like.

The "lower alkyl group" is a straight chain or branched chain or cyclic alkyl group having a carbon number of 1 to 6. For example, methyl group, ethyl group, n-propyl group, n-butyl group, n-pentyl group, n-hexyl group, isopropyl group, isobutyl group, sec-butyl group, tert-butyl group, isopentyl group, tert-pentyl group, neopentyl group, 2-pentyl group, 3-pentyl group, 2-hexyl group, cyclopropyl group, cyclobutyl group, cyclopentyl group and the like can be mentioned.

The "halogen atom" is, for example, a fluorine atom, a chlorine atom, a bromine atom, an iodine atom and the like.

The "C₃₋₈ cycloalkane ring" is a cycloalkane ring having a carbon number of 3 to 8. For example, cyclopropane ring, cyclobutane ring, cyclopentane ring, cyclohexane ring, cycloheptane ring, and cyclooctane ring can be mentioned.

The "lower acyl group" is an acyl group having a straight chain or branched chain or cyclic alkyl group or alkenyl group having a carbon number of 1 to 6. For example, acetyl group, propionyl group, butyryl group, isobutyryl group, valeryl group, isovaleryl group, pivaloyl group, hexanoyl group, acryloyl group, methacryloyl group, crotonoyl group, isocrotonoyl group, cyclopropanoyl group, cyclobutanoyl group, cyclopentanoyl group and cyclohexanoyl group and the like can be mentioned.

The "lower alkoxyl group" is an alkoxyl group having a straight chain or branched chain or cyclic alkyl group having a carbon number of 1 to 6. For example, methoxy group, ethoxy group, n-propoxy group, n-butoxy group, n-pentyloxy group, n-hexyloxy group, isopropoxy group, isobutoxy group, sec-butoxy group, tert-butoxy group, cyclopropyloxy group, cyclobutyloxy group, cyclopentyloxy group and cyclohexyloxy group can be mentioned.

The "lower alkylthio group" is an alkylthio group having a straight chain or branched chain or cyclic alkyl group having a carbon number of 1 to 6. For example, methylthio group, ethylthio group, n-propylthio group, isopropylthio group, n-butylthio group, isobutylthio group, sec-butylthio group, tert-butylthio group, cyclopropylthio group, cyclobutylthio group, cyclopentylthio group, cyclobutylthio group and the like can be mentioned.

In the aforementioned formulas, preferable embodiments are as follows.

R² and R³ are the same or different and each is independently a hydrogen atom or a lower alkyl group optionally having a substituent(s). Preferably, R² and R³ are hydrogen atoms.

R⁶ and R⁷ are the same or different and each is independently a hydrogen atom or a lower alkyl group optionally having a substituent(s), or R⁶ and R⁷ form a C₃₋₈ cycloalkane ring together with a carbon atom bonded thereto. Preferably, R⁶ and R⁷ are the same and are methyl groups, ethyl groups or propyl groups, or R⁶ and R⁷ form a cyclopropane ring, a cyclobutane ring or a cyclopentane ring together with a carbon atom bonded thereto. More preferably, R⁶ and R⁷ are methyl groups.

R⁴ is a lower alkyl group. Preferably, R⁴ is a methyl group, an ethyl group, an isopropyl group or an n-butyl group. More preferably, R⁴ is a methyl group.

R⁵ is a lower alkyl group. Preferably, R⁵ is a tert-butyl group.

R⁸, R⁹, R¹⁰ and R¹¹ are the same or different and each is a hydrogen atom or a halogen atom. Preferably, R⁸, R⁹ and R¹¹ are hydrogen atoms, and R¹⁰ is a fluorine atom.

X is a halogen atom. Preferably, X is a chlorine atom.

When the compound of the present invention can form a salt, a pharmaceutically acceptable salt is preferable. Examples of such pharmaceutically acceptable salt for a compound having an acidic group such as a carboxyl group and the like include ammonium salt, salts with alkali metals such as sodium, potassium and the like, salts with alkaline earth metals such as calcium, barium and the like, magnesium salt, aluminum salt, zinc salt, salts with organic amines such as triethylamine, ethanolamine, morpholine, pyrrolidine, piperidine, piperazine, dicyclohexylamine and the like, and salts with basic amino acids such as arginine, lysine and the like. Examples of such pharmaceutically acceptable salt for a compound having a basic group include salts with inorganic acids such as hydrochloric acid, sulfuric acid, phosphoric acid, nitric acid, hydrobromic acid and the like, salts with organic carboxylic acids such as acetic acid, citric acid, benzoic acid, maleic acid, fumaric acid, tartaric acid, succinic acid, trifluoroacetic acid, tannic acid, butyric acid, hibenzic acid, pamoic acid, enanthic acid, decanoic acid, teoclic acid, salicylic acid, lactic acid, oxalic acid, mandelic acid, malic acid and the like, and salts with organic sulfonic acids such as methanesulfonic acid, benzenesulfonic acid, p-toluenesulfonic acid and the like.

As a salt used in the present invention, a chemically acceptable salt can be mentioned in addition to those recited as the above-mentioned pharmaceutically acceptable salt, and a salt with a chemically acceptable acid and a salt with a chemically acceptable base are included.

As a salt with a chemically acceptable acid to be used in the present invention, salts with inorganic acids (e.g., hydrochloric acid, sulfuric acid, phosphoric acid, nitric acid, hydrobromic acid, etc.), organic carboxylic acids (e.g., carbonic acid, acetic acid, citric acid, benzoic acid, maleic acid, fumaric acid, tartaric acid, succinic acid, trifluoroacetic acid, tannic acid, butyric acid, decanoic acid, salicylic acid, lactic acid, oxalic acid, mandelic acid, malic acid, etc.), organic sulfonic acids (e.g., methanesulfonic acid, p-toluenesulfonic acid, benzenesulfonic acid, etc.) and the like can be mentioned.

As a salt with a chemically acceptable base, alkali metal salts (e.g., sodium salt, potassium salt, lithium salt, etc.), alkaline earth metal salts (e.g., calcium salt, barium salt, etc.), metal salts (e.g., magnesium salt, aluminum salt, etc.) and the like can be mentioned.

The compound of the present invention also includes solvates of the compound such as hydrate, alcohol adduct and the like. As the alcohol adduct, methanol solvate, ethanol solvate, isopropyl alcohol solvate and the like can be mentioned.

A production method of a heteroarylcarboxylic acid ester derivative represented by the formula (9) is shown below.

The present invention is a method of producing a compound represented by the formula (9) or a chemically acceptable salt thereof, using the following steps (a) to (f),

(a) synthesizing a diester represented by the formula (3) by reacting a compound represented by the formula (1) with a compound represented by the formula (2):

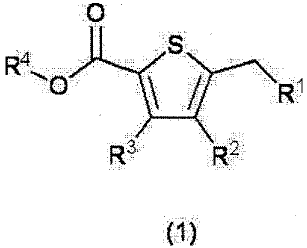

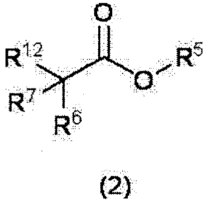

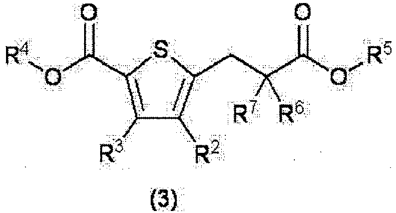

(b) synthesizing an ester represented by the formula (4) by deprotecting the diester of the formula (3):

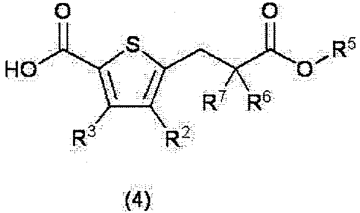

(c) synthesizing an ester represented by the formula (4) by reacting a compound represented by the formula (5) with a compound represented by (2): 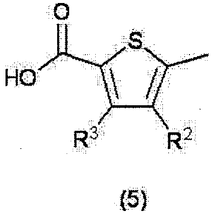 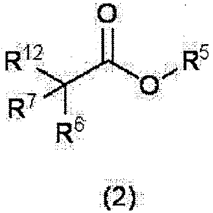 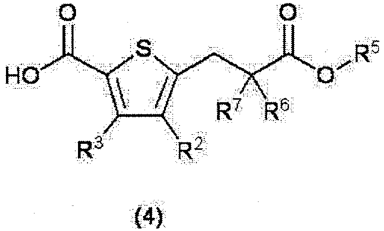 (d) reacting the compound represented by the formula (4) with a halogenating agent to give an acid halide represented by the formula (6): 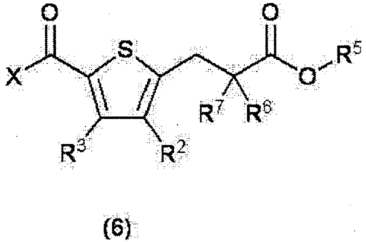 (e) reacting the compound represented by the formula (6) with a compound represented by the formula (7) to give a compound represented by the formula (8) or a chemically acceptable salt thereof: 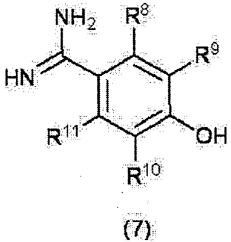 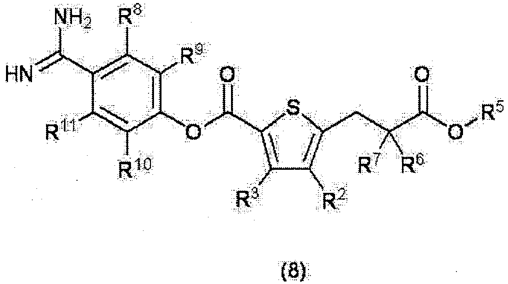 (f) deprotecting the compound represented by the formula 8) under acidic conditions to give a heteroarylcarboxylic acid ester derivative represented by the formula (9), or a chemically acceptable salt thereof:

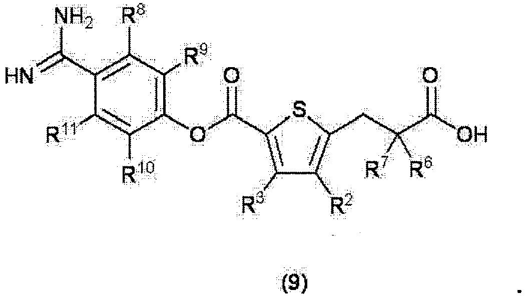

A preferable embodiment is described in more detail in the following.

### Step (a)

This reaction is preferably performed in the presence of a base.

As the base to be used for the reaction, (A) an alkyllithium, or (B) a lithium complex prepared from an organic amine such as diisopropylamine, diisopropylethylamine and the like, and an alkyllithium can be mentioned. Use of (A) n-butyllithium, or (B) a lithium complex prepared from an organic amine such as diisopropylamine, diisopropylethylamine and the like, and n-butyllithium is preferable. The amount of the base to be used is preferably about 1.0 mol to about 2.0 mol, per 1 mol of the compound of the formula (1).

As the reaction solvent, ethers and hydrocarbons can be used, of which tetrahydrofuran is preferable.

The reaction temperature is preferably -78°C to 0°C. The reaction time is generally about 30 min.

### Step (b)

As a reagent to be used for the deprotection, a metal hydroxide such as sodium hydroxide and the like is preferable. As the metal hydroxide, alkali metal hydroxide such as sodium hydroxide, lithium hydroxide, potassium hydroxide and the like can be mentioned. The amount of the metal hydroxide to be used is preferably about 1.0 mol to about 2.0 mol, per 1 mol of the diester of the formula (3).

As the reaction solvent, a mixed solvent of an alcohol and water is preferable. As the alcohol, methanol, ethanol and the like can be mentioned.

The reaction temperature is preferably 10°C to 40°C. The reaction time is generally about 12 hr to about 24 hr.

### Step (c)

This reaction is preferably performed in the presence of a base.

As the base to be used, a lithium complex prepared from an organic amine such as diisopropylamine, 2,2,6,6-tetramethylpiperidine and the like and n-butyllithium, and an alkali metal hydride are preferable. As the lithium complex, lithium diisopropylamine (LDA) is preferable. As the alkali metal hydride, sodium hydride (NaH) is preferable. The amount of the lithium complex to be used is preferably about 1.0 mol to about 1.2 mol per 1 mol of the compound of the formula (5), and the amount of the alkali metal hydride (e.g., sodium hydride) is also preferably about 1.0 mol to about 1.2 mol, per 1 mol of the compound of the formula (5).

As the reaction solvent, ethers and hydrocarbons can be used, of which tetrahydrofuran is preferable.

The reaction temperature is preferably -78°C to 0°C. The reaction time is generally about 30 min.

### Step (d)

As the reaction solvent for producing an acid halide, an acetic acid ester is preferable, and as the halogenating agent, thionyl chloride or oxalyl chloride is preferable. As the additive, N,N-dimethylformamide (DMF) and N-methylpyrrolidone (NMP) are preferable.

The amount of the halogenating agent to be used is preferably about 1.0 mol to about 1.5 mol, per 1 mol of the compound of the formula (4).

The reaction temperature is between 0°C and 45°C, preferably 10°C to 30°C. The reaction time is generally about 30 min to about 2 hr.

### Step (e)

A diester derivative represented by the formula (8) is obtained by reacting acid halide (6) obtained in step (d) with amidinophenol derivative (7) preferably in the presence of a base. As the reaction solvent, acetonitrile is preferable.

As the base, organic bases such as pyridine, triethylamine, diisopropylethylamine, lutidine and the like can be mentioned. As the base, pyridine is preferable. The amount of the base to be used is generally about 2.0 mol to about 3.0 mol, per 1 mol of acid halide (6).

The amount of the amidinophenol derivative (7) to be used is generally preferably about 1.0 mol to about 1.2 mol, per 1 mol of the acid halide (6).

The reaction temperature is between -60°C and 30°C, preferably -25°C to 10°C. The reaction time is generally about 30 min.

A diester derivative (8) can be crystallized as a TFA salt from the reaction system by adding trifluoroacetic acid (TFA) and water dropwise to the reaction mixture, and can be isolated and purified by filtration separation. The amount of TFA to be used is generally about 2.0 mol to about 3.0 mol, per 1 mol of acid halide (6).

The temperature of dropwise addition and crystallization is preferably 0°C to 20°C. The crystallization time is generally about 2 to about 24 hr.

The precipitated diester derivative (8) or a salt thereof can be conveniently isolated and purified by filtration.

### Step (f)

A heteroarylcarboxylic acid ester derivative represented by the formula (9) is obtained by deprotecting the diester derivative (8) obtained in step (e) under acidic conditions.

As the acid to be used for the reaction, HCl, HBr, sulfuric acid, methanesulfonic acid, trifluoroacetic acid and the like can be mentioned. Preferred is HCl.

As the reaction solvent, 1,4-dioxane and acetone are preferable, and as the additive, water is preferably added.

The reaction temperature is preferably 10°C to 60°C. The reaction time is generally about 6 hr to about 24 hr.

By the reaction, a heteroarylcarboxylic acid ester derivative can be crystallized and isolated by filtration.

The term "reaction system" means a reaction mixture after performing a reaction or a reaction mixture obtained by filtering an insoluble material (e.g., catalyst and the like) off from a reaction mixture.

The compounds of the formula (1) and the formula (5) can be produced by a method known per se.

The present invention also provides the following intermediate to be used for the method of the present invention.

A compound represented by the following formula (20), or a chemically acceptable salt thereof: 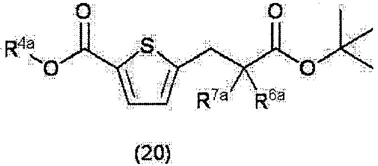
wherein R^{4a} is a methyl group, an ethyl group, an isopropyl group or an n-butyl group, and
R^{6a} and R^{7a} are the same and are methyl groups or ethyl groups, or R^{6a} and R^{7a} form a cyclopropane ring, a cyclobutane ring or a cyclopentane ring together with a carbon atom bonded thereto.

As a preferable embodiment of the compound represented by the formula (20), the following compounds can be mentioned.

Compounds having the following structures, or chemically acceptable salts thereof.

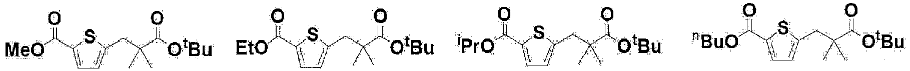

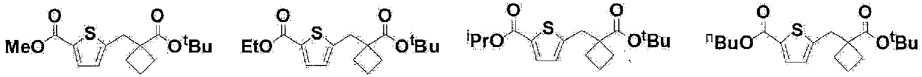

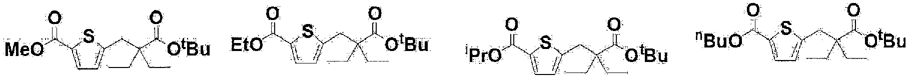

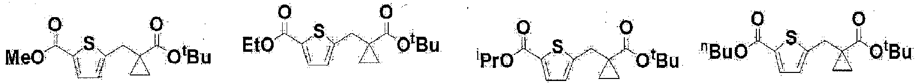

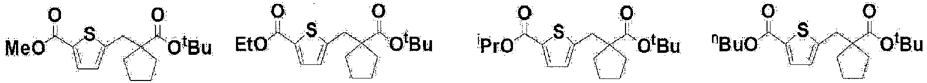

Compounds having the following structures, or chemically acceptable salts thereof.

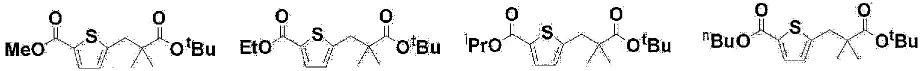

Compounds having the following structures, or chemically acceptable salts thereof.

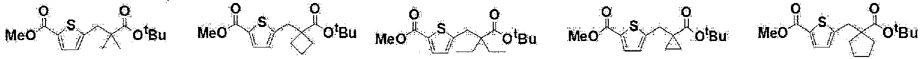

A compound represented by the following formula (10), or a chemically acceptable salt thereof.

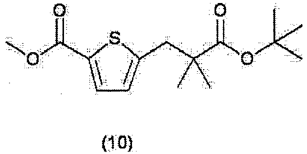

A compound represented by the following formula (21), or a chemically acceptable salt thereof. 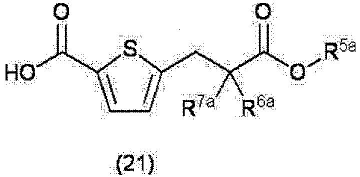
wherein R^{5a} is a methyl group, an ethyl group, an isopropyl group, a tert-butyl group or an n-butyl group,
R^{6a} and R^{7a} are the same and are methyl groups or ethyl groups, or R^{6a} and R^{7a} form a cyclopropane ring, a cyclobutane ring or a cyclopentane ring together with a carbon atom bonded thereto.

As a preferable embodiment of the compound represented by the formula (21), the following compounds can be mentioned.

Compounds having the following structures, or chemically acceptable salts thereof.

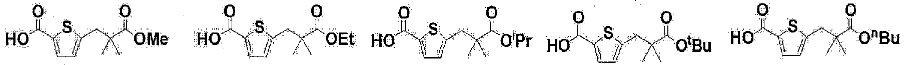

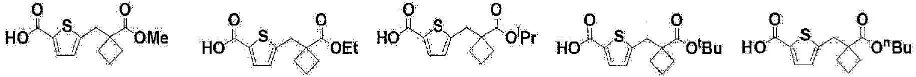

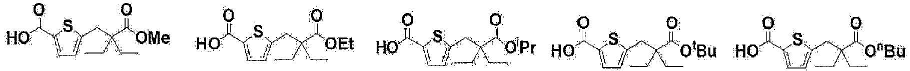

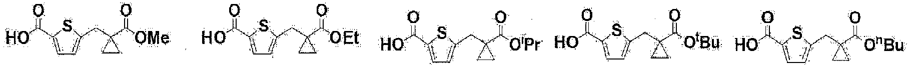

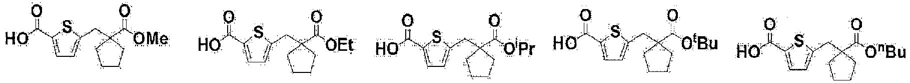

Compounds having the following structures, or chemically acceptable salts thereof.

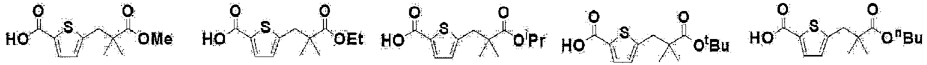

Compounds having the following structures, or chemically acceptable salts thereof.

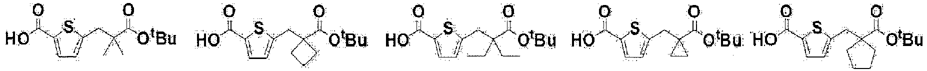

A compound represented by the following formula (11), or a chemically acceptable salt thereof.

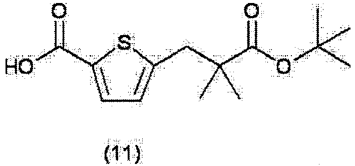

### Examples

While the present invention is explained in detail in the following by referring to Examples, the present invention is not limited to the following Examples.

### (Analysis conditions)

The analysis in the following Examples was performed using the following measuring apparatus and according to a conventional method.

### (1) ¹H-NMR

apparatus: Avance III 400, manufactured by Burker

### (2) high performance liquid chromatography (HPLC)

HPLC: LC-2010AHT (manufactured by Shimadzu Corporation)
column used: InertSustain C18 ϕ 4.6 mmx150 mm, particle size 3 µm (manufactured by GL-Sciences)
detection wavelength: UV 254 nm
column temperature: 40°C
injection volume: 10 µL
analysis time: 18 min
flow rate: 1.5 mL/min
eluent: solution A: water/trifluoroacetic acid mixed solution
(1000:1)
solution B: acetonitrile/trifluoroacetic acid mixed solution
(1000:1)
gradient: 0 min: 20%B, 0→13 min: 20→90%B, 13→15 min: 90%B, 15→15.1 min: 90→20%B, 15.1→18 min: 20%B

The yield in each step of synthesis is a value calculated based on the HPLC area value of the standard product.

### (Example 1)

### Synthesis of 5-(2-tert-butoxycarbonyl-2-methylpropyl)thiophene-2-carboxylic acid methyl ester

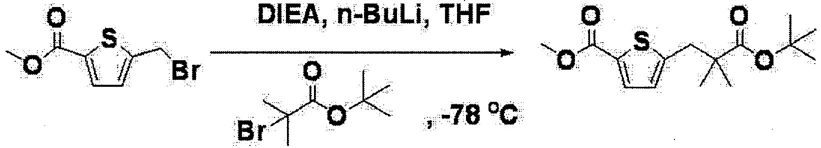

To a solution of diisopropylethylamine (46.8 mL, 1.87 eq) in tetrahydrofuran (THF, 169 mL) was added dropwise n-BuLi (95.1 mL, 1.75 eq, 2.65 M in hexane) at -78°C over 5 min, and the mixture was directly stirred for 15 min. Thereafter, 2-bromo-2-methylpropanoic acid tert-butyl ester (43.8 mL, 1.63 eq) was added dropwise, and the mixture was directly stirred for 30 min. Thereafter, a solution of 5-bromomethylthiophene-2-carboxylic acid methyl ester in cyclopentyl methyl ether (CPME) (gross 95.43 g, net 33.86 g, 144.0 mol) was added and the mixture was stirred for 1 hr. Water (169 mL) was added, and the mixture was extracted with heptane (339 mL), washed with a mixture of ethanol (112 mL) and water (223 mL), and concentrated to give the title compound (gross 59.28 g, net 37.10 g) (yield 86.3%).
¹H NMR (400 MHz, Chloroform-d) δ 7.62 (d, J = 3.8 Hz, 1H), 6.79 (d, J = 3.8 Hz, 1H), 3.85 (s, 3H), 3.04 (s, 2H), 1.45 (s, 9H), 1.18 (s, 6H).

### (Example 2)

### Synthesis of 5-(2-tert-butoxycarbonyl-2-methylpropyl)thiophene-2-carboxylic acid

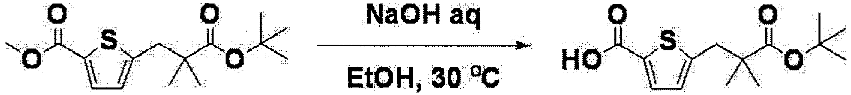

To 5-(2-tert-butoxycarbonyl-2-methylpropyl)thiophene-2-carboxylic acid methyl ester (gross 59.28 g, net 37.10 g, 124.3 mmol) were added water (80.2 mL) and ethanol (111.3 mL), and 6 N NaOH aqueous solution (31.1 mL, 1.5 eq) was added and the mixture was stirred at 30°C for 16 hr. After completion of the reaction, and the mixture was neutralized with 6 N HCl (31.1 mL, 1.5 eq), and extracted with cyclohexane (371 mL). After washing with a mixture of ethanol (122 mL) and water (245 mL), activated carbon (1.86 g) was added. After stirring at 25°C for 22 hr, activated carbon was removed by celite filtration, and concentration under reduced pressure and dropwise addition of heptane (371 mL) were performed. The mixture was heated to 60°C, cooled to 20°C, stirred for 30 min, and then cooled to 5°C. After stirring for 20 hr, the precipitated crystals were collected by filtration, washed with cold heptane (74.2 mL, 10°C), and dried under reduced pressure at 50°C to give the title compound (22.74 g) (yield 64.3%).
¹H NMR (400 MHz, Chloroform-d) δ 7.70 (d, J = 3.8 Hz, 1H), 6.83 (d, J = 3.8 Hz, 1H), 3.06 (s, 2H), 1.46 (s, 9H), 1.19 (s, 6H).

### (Example 3)

### Synthesis of 5-(2-tert-butoxycarbonyl-2-methylpropyl)thiophene-2-carboxylic acid

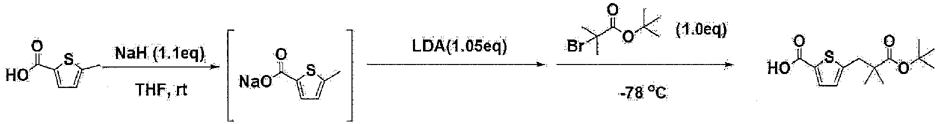

A mineral oil dispersion of NaH (NaH concentration 60 mass%, 840 mg) was suspended in THF (19.9 mL), and a solution of 5-methylthiophene-2-carboxylic acid (2.84 g, 20.0 mmol) in THF (11.3 mL) was added dropwise at room temperature. The mixture was heated to 60°C and stirred for 10 min, cooled again to room temperature, and diisopropylamine (3.1 mL, 1.1 eq) was added. After cooling to 0°C, n-BuLi (10.5 mL, 1.05 eq, in 2.0 M cyclohexane) was added dropwise, and the mixture was directly stirred for 10 min. After cooling to -78°C, 2-bromoisobutyric acid tert-butyl ester (3.72 mL, 20.0 mol) was added and the mixture was stirred for 30 min. After warming to 0°C, 6 N HCl (11.7 mL, 3.5 eq) and heptane (28.4 mL) were added, and the mixture was stirred at 0°C for 1 hr. The resulting solid was collected by filtration, the mother liquor was washed with water (17 mL), and the solvent (about 25 mL) was evaporated under reduced pressure. Heptane (28.4 mL) was added, and the mixture was washed with methanol/water =2/3 (volume ratio, 17 mL) and methanol/water =7/8 (volume ratio, 17 mL), and the solvent (about 60 mL) was evaporated under reduced pressure. Heptane (28.4 mL) and activated carbon (142 mg) were added, and the mixture was heated to 60°C and stirred for 1 hr. Activated carbon was removed by celite filtration, and the filtrate was concentrated under reduced pressure to give about 30 mL of a solution, which was stirred at room temperature for 1 hr. Thereafter, the solution was cooled to 10°C, and stirred for 16 hr. The precipitated crystals were collected by filtration, washed with cold heptane (2.8 mL, 10°C), and dried under reduced pressure at 40°C for 6 hr to give the title compound (2.44 g) (yield 42.9%).
¹H NMR (400 MHz, Chloroform-d) δ 7.71 (d, J = 3.8 Hz, 1H), 6.83 (d, J = 3.8 Hz, 1H), 3.06 (s, 2H), 1.46 (s, 9H), 1.19 (s, 6H).

### (Example 4)

### Synthesis of 5-(2-tert-butoxycarbonyl-2-methylpropyl)thiophene-2-carboxylic acid 4-carbamimidoyl-2-fluorophenyl ester

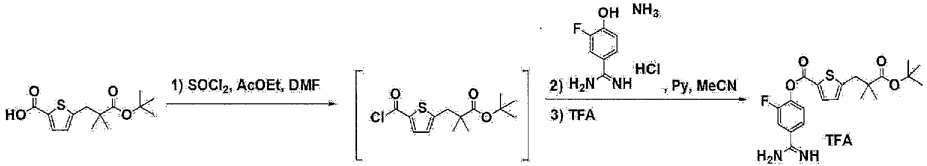

To a solution of 5-(2-tert-butoxycarbonyl-2-methylpropyl)thiophene-2-carboxylic acid (2.84 g) in ethyl acetate (AcOEt, 11.4 mL) were added dropwise N,N-dimethylformamide (DMF, 284 µL) and thionyl chloride (1.1 mL, 1.5 eq) at 25°C, and the mixture was stirred at 25°C for 30 min. The production of acid chloride was confirmed, and the reaction solution was concentrated, and acetonitrile (MeCN, 8.5 mL) was added. This solution was added dropwise to a suspension of ammonia-hydrogen chloride mixture of 3-fluoro-4-hydroxybenzamidine (2.26 g, 1.1 eq) in acetonitrile (17.0 mL) at 0°C. After the completion of the dropwise addition, pyridine (2.0 mL, 2.5 eq) was added. After 30 min, water (25.6 mL) and trifluoroacetic acid (TFA, 1.9 mL, 2.5 eq) were added dropwise at 15°C. After crystallization, water (25.6 mL) was added, and the mixture was stirred at 10°C overnight. The precipitated crystals were collected by filtration, washed with water (5.7 mL), and dried under reduced pressure at 50°C to give the title compound (4.83 g) (yield 90.4%).
¹H NMR (400 MHz, DMSO-d₆) δ 9.57 (s, 2H), 9.45 (s, 2H), 8.02 - 7.91 (m, 2H), 7.81 - 7.73 (m, 2H), 7.09 (d, J = 3.8 Hz, 1H), 3.13 (s, 2H), 1.42 (s, 9H), 1.15 (s, 6H).

### (Example 5)

### Synthesis of 3-[5-(4-carbamimidoyl-2-fluorophenoxy)carbonyl-2-thienyl]-2,2-dimethylpropanoic acid hydrochloride

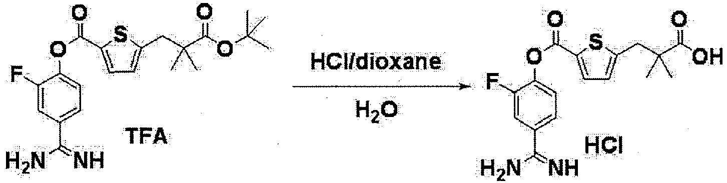

To a suspension of 5-(2-tert-butoxycarbonyl-2-methylpropyl)thiophene-2-carboxylic acid 4-carbamimidoyl-2-fluorophenyl ester (3.21 g) in 1,4-dioxane (9.6 mL) were added 4 M HCl/1,4-dioxane (9.6 mL) and Milli Q water (96 µL), and the mixture was stirred at 50°C for 15 hr. After the reaction, the slurry was filtered, and the solid collected by filtration was washed with acetone (6.4 mL), and dried under reduced pressure at 50°C to give the title compound (2.26 g) (yield 92.1%).
¹H NMR (400 MHz, DMSO-d₆) 5 12.58 (s, 1H), 9.62 (s, 2H), 9.47 (s, 2H), 8.08 - 7.90 (m, 2H), 7.87 - 7.71 (m, 2H), 7.09 (d, J = 3.8 Hz, 1H), 3.14 (s, 2H), 1.17 (s, 6H).

### (Example 6)

### Purification of 3-[5-(4-carbamimidoyl-2-fluorophenoxy)carbonyl-2-thienyl]-2,2-dimethylpropanoic acid hydrochloride

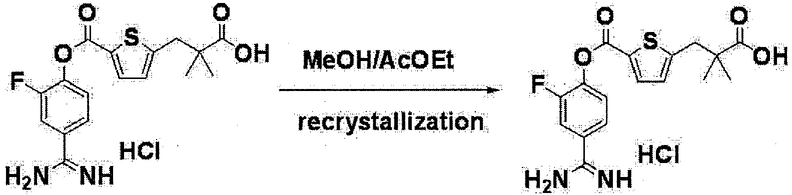

The crude crystals (1.50 g) obtained in Example 5 were dissolved by adding a mixed solvent (21.0 mL) of MeOH/AcOEt=1/1, Milli Q water (45 µL) and methanol (1.5 mL). Thereafter, concentration substitution with ethyl acetate was performed (total 37.5 mL of ethyl acetate was used). The precipitated crystals were collected by filtration, washed with ethyl acetate (6.0 mL), and dried under reduced pressure at 50°C to give the title compound (1.42 g) (yield 96.6%).
¹H NMR (400 MHz, DMSO-d₆) δ 12.56 (s, 1H), 9.54 (s, 2H), 9.36
(s, 2H), 8.10 - 7.87 (m, 2H), 7.84 - 7.68 (m, 2H), 7.09 (d, J = 3.8 Hz, 1H), 3.14 (s, 2H), 1.16 (s, 6H).

### Industrial Applicability

Using the production method of the present invention, the object compound, a heteroarylcarboxylic acid ester derivative, can be produced in a high yield and with high purity. The compound of the formula (20), the compound of the formula (10), the compound of the formula (21), the compound of the formula (11), the compound of the formula (8), and the compound of the formula (15) are useful as intermediates for producing a heteroarylcarboxylic acid ester derivative (9).

This application is based on patent application No. 2014-48091 filed in Japan, the entire contents of which are incorporated by reference herein.

## Claims

1. A production method of an ester derivative represented by the formula (4), or a chemically acceptable salt thereof: 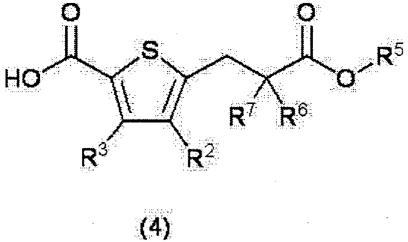
wherein R² and R³ are the same or different and each is independently a hydrogen atom or a lower alkyl group,
R³ is a tert-butyl group,
R⁶ and R⁷ are the same or different and each is independently a hydrogen atom or a lower alkyl group optionally having a substituent(s) , or R⁶ and R⁷ form a C₃₋₈ cycloalkane ring together with a carbon atom bonded thereto,
the method comprising the following steps (g) and (h):
(g) reacting a compound represented by the formula (1) and a compound represented by the formula (2) in the presence of (A) an alkyllithium or (B) a lithium complex prepared from an organic amine and an alkyllithium to give a compound represented by the formula (3): 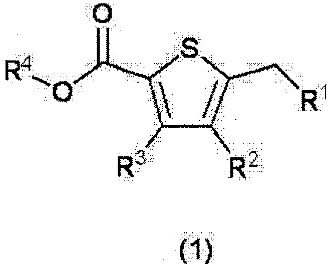 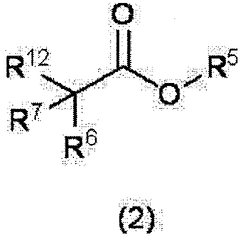 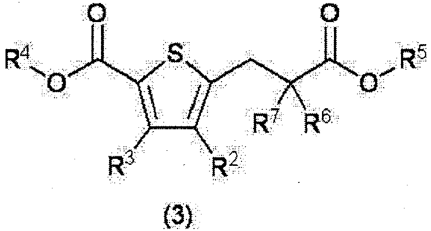
wherein R¹ is a halogen atom,
R⁴ is a methyl group, an ethyl group, an isopropyl group or an n-butyl group,
R¹² is a hydrogen atom or a halogen atom, and
other symbols are as defined above, and
(h) hydrolyzing the compound represented by the formula (3) with a metal hydroxide in a solvent containing water and an alcohol to give an ester derivative represented by the formula (4), or a chemically acceptable salt thereof.

2. The production method according to claim 1, wherein the alkyllithium is n-butyllithium.

3. A production method of an ester derivative represented by the formula (11), or a chemically acceptable salt thereof: 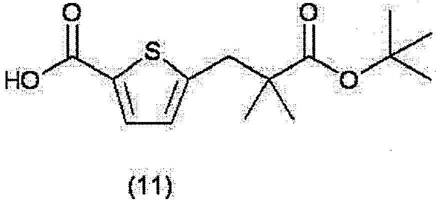 the method comprising the following steps (k) and (1):
(k) reacting a compound represented by the formula (12) with a compound represented by the formula (13) in the presence of (A) n-butyllithium or (B) a lithium complex prepared from an organic amine selected from diisopropylamine and diisopropylethylamine, and n-butyllithium to give a compound represented by the formula (10): 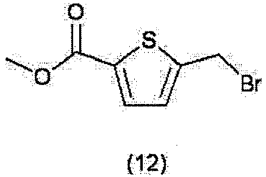 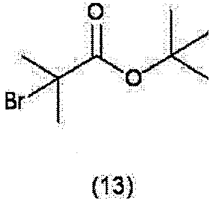 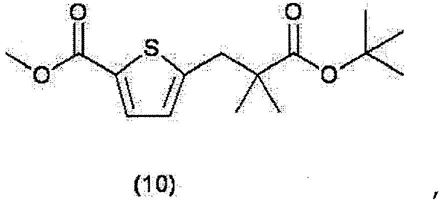 and
(1) hydrolyzing the compound represented by the formula (10) with a metal hydroxide selected from sodium hydroxide, lithium hydroxide and potassium hydroxide in a solvent containing water and an alcohol to give an ester derivative represented by the formula (11), or a chemically acceptable salt thereof.

4. A production method of an ester derivative represented by the formula (4), or a chemically acceptable salt thereof: 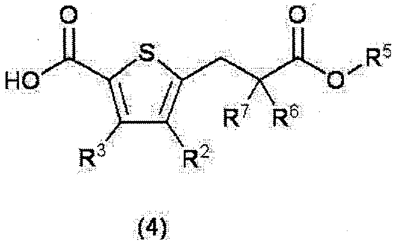
wherein R² and R³ are the same or different and each is independently a hydrogen atom or a lower alkyl group optionally having a substituent(s),
R⁵ is a lower alkyl group, and
R⁶ and R⁷ are the same or different and each is independently a hydrogen atom or a lower alkyl group optionally having a substituent(s), or R⁶ and R⁷ form a C₃₋₈ cycloalkane ring together with a carbon atom bonded thereto,
the method comprising step (m):
(m) reacting a compound represented by the formula (5) with a compound represented by, the formula (2) using a base to give an ester derivative represented by the formula (4), or a chemically acceptable salt thereof: 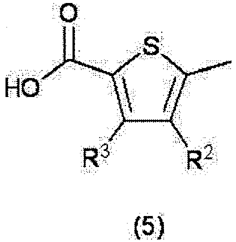 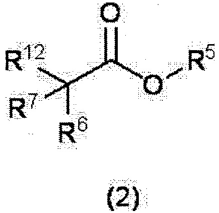
wherein R¹² is a halogen atom, and
other symbols are as defined above.

5. A production method of an ester derivative represented by the formula (11), or a chemically acceptable salt thereof: 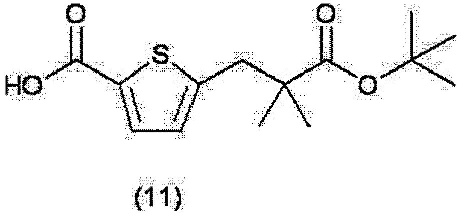 the method comprising step (n):
(n) reacting a compound represented by the formula (14) with a compound represented by the formula (13) using sodium hydride and lithium diisopropylamide to give an ester derivative represented by the formula (11), or a chemically acceptable salt thereof:

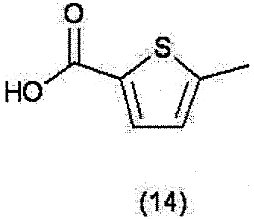

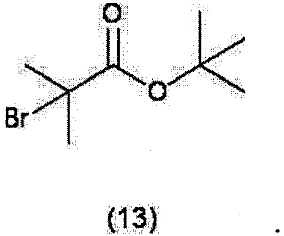

6. A production method of a heteroarylcarboxylic acid ester derivative represented by the formula (9), or a chemically acceptable salt thereof: 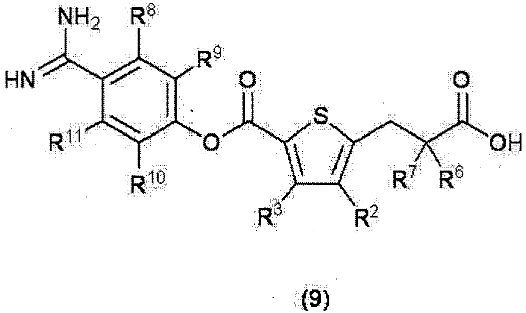
wherein R² and R³ are the same or different and each is independently a hydrogen atom or a lower alkyl group optionally having a substituent(s),
R⁶ and R⁷ are the same or different and each is independently a hydrogen atom or a lower alkyl group optionally having a substituent(s), or R⁶ and R⁷ form a C₃₋₈ cycloalkane ring together with a carbon atom bonded thereto, and
R⁸, R⁹, R¹⁰ and R¹¹ are the same or different and each is independently a hydrogen atom or a halogen atom,
the method comprising the following steps (o) to (q):
(o) reacting the compound represented by the formula (4) with a halogenating agent to give an acid halide represented by the formula (6): 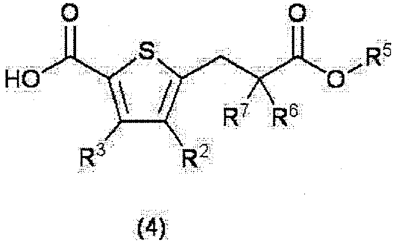 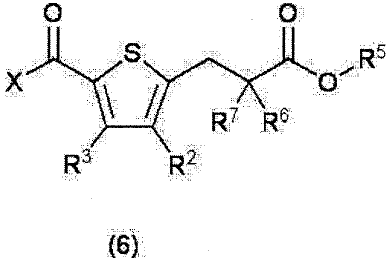
wherein R⁵ is a lower alkyl group,
X is a halogen atom, and
other symbols are as defined above,
(p) reacting the acid halide represented by the formula (6) with a compound represented by the formula (7) to give a compound represented by the formula (8), or a chemically acceptable salt thereof: 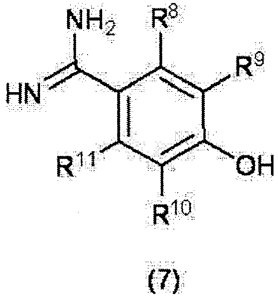 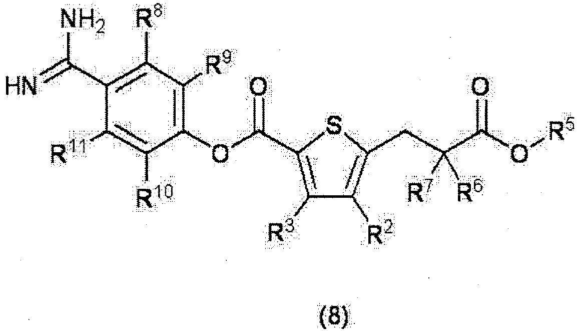 wherein the symbols are as defined above, and
(q) deprotecting the compound represented by the formula (8), or a chemically acceptable salt thereof under acidic conditions to give a heteroarylcarboxylic acid ester derivative represented by the formula (9), or a chemically acceptable salt thereof.

7. A production method of a heteroarylcarboxylic acid ester derivative represented by the formula (9), or a chemically acceptable salt thereof: 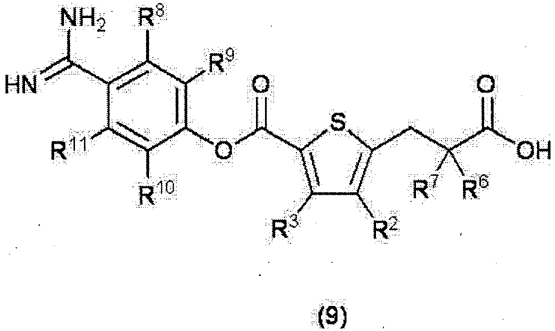
wherein R² and R³ are the same or different and each is independently a hydrogen atom or a lower alkyl group optionally having a substituent(s),
R⁶ and R⁷ are the same or different and each is independently a hydrogen atom or a lower alkyl group optionally having a substituent(s), or R⁶ and R⁷ form a C₃₋₈ cycloalkane ring together with a carbon atom bonded thereto, and
R⁸, R⁹, R¹⁰ and R¹¹ are the same or different and each is independently a hydrogen atom or a halogen atom,
the method comprising the following steps (r) to (t):
(r) reacting a compound represented by the formula (4) with thionyl chloride or oxalyl chloride to give an acid chloride represented by the formula (16): 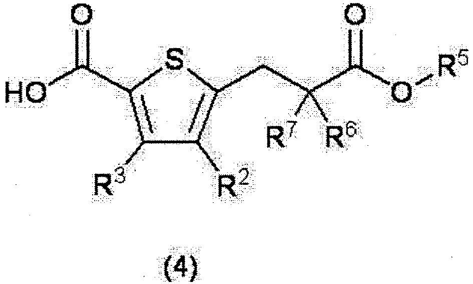 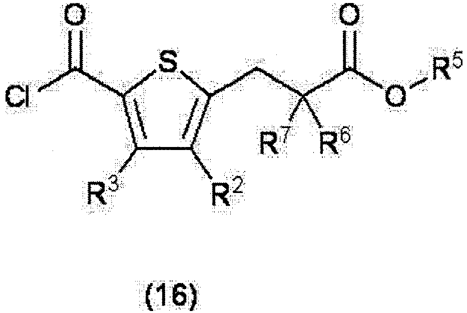 wherein R⁵ is a lower alkyl group, and
other symbols are as defined above,
(s) reacting the acid chloride represented by the formula (16) with a compound represented by the formula (7) in the presence of an organic base to give a compound represented by the formula (8), or a chemically acceptable salt thereof: 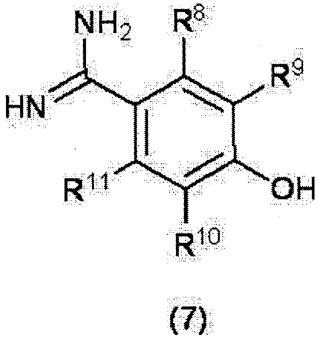 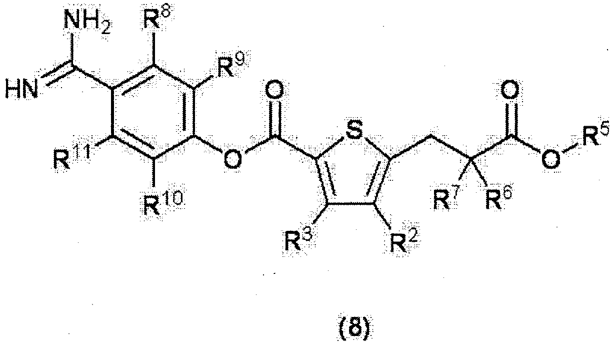 wherein each symbol is as defined above, and
(t) deprotecting the compound represented by the formula (8), or a chemically acceptable salt thereof with HCl, HBr, sulfuric acid, methanesulfonic acid or trifluoroacetic acid to give a heteroarylcarboxylic acid ester derivative represented by the formula (9), or a chemically acceptable salt thereof.

8. A production method of a heteroarylcarboxylic acid ester derivative represented by the formula (17), or a chemically acceptable salt thereof: 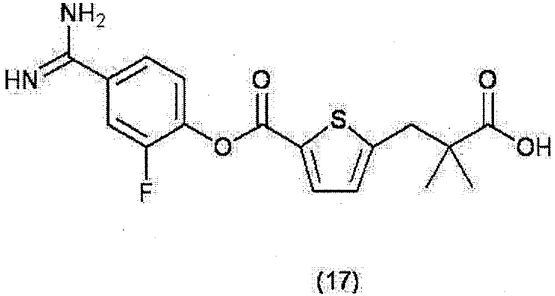 the method comprising the following steps (u) to (w):
(u) reacting a compound represented by the formula (11) with thionyl chloride to give an acid chloride represented by the formula (18): 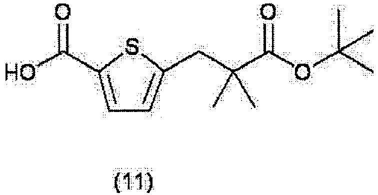 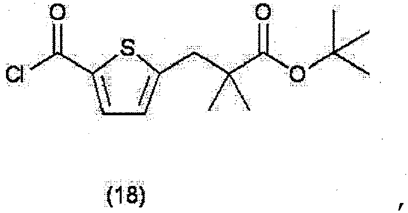
(v) reacting the acid chloride represented by the formula (18) with a compound represented by the formula (19) in the presence of pyridine to give a compound represented by the formula (15), or a chemically acceptable salt thereof: 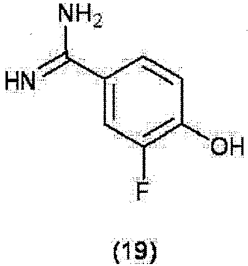 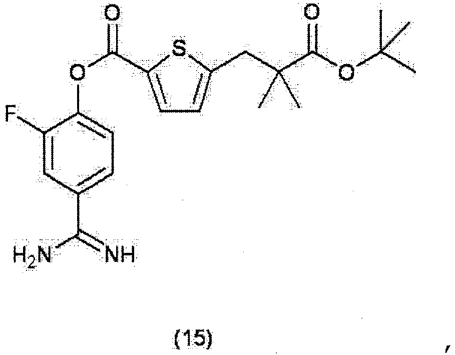 and
(w) deprotecting a compound represented by the formula (15), or a chemically acceptable salt thereof in the presence of HCl to give a heteroarylcarboxylic acid ester derivative represented by the formula (17), or a chemically acceptable salt thereof.

9. A compound represented by the following formula (20), or a chemically acceptable salt thereof: 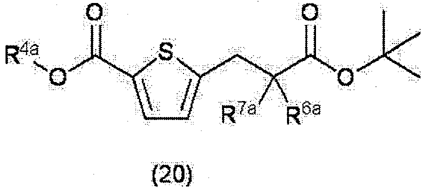 wherein R^{4a} is a methyl group, an ethyl group, an isopropyl group or an n-butyl group,
R^{6a} and R^{7a} are the same and are methyl groups or ethyl groups, or R^{6a} and R^{7a} form a cyclopropane ring, a cyclobutane ring or a cyclopentane ring together with a carbon atom bonded thereto.

10. The compound according to claim 9, wherein R^{6a} and R^{7a} are methyl groups, or a chemically acceptable salt thereof.

11. The compound according to claim 9, wherein R^{4a} is a methyl group, or a chemically acceptable salt thereof.

12. A compound represented by the following formula (10), or a chemically acceptable salt thereof:

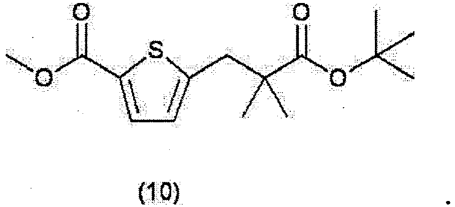

13. A compound represented by the following formula (21), or a chemically acceptable salt thereof: 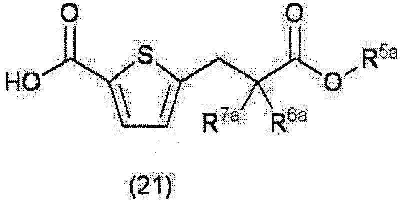
wherein R^{5a} is a methyl group, an ethyl group, an isopropyl group, a tert-butyl group or an n-butyl group,
R^{6a} and R^{7a} are the same and are methyl groups or ethyl groups,
or R^{6a} and R^{7a} form a cyclopropane ring, a cyclobutane ring or a cyclopentane ring together with a carbon atom bonded thereto.

14. The compound according to claim 13, wherein R^{6a} and R^{7a} are methyl groups, or a chemically acceptable salt thereof.

15. The compound according to claim 13, wherein R^{5a} is a tert-butyl group, or a chemically acceptable salt thereof.

16. A compound represented by the following formula (11), or a chemically acceptable salt thereof:

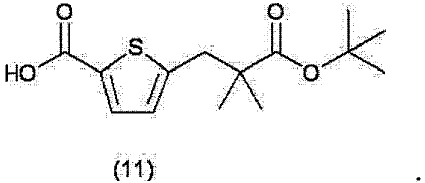

17. A compound represented by the following formula (8), or a chemically acceptable salt thereof 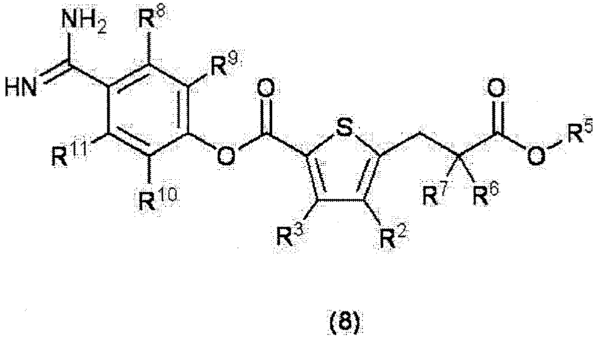
wherein R² and R³ are the same or different and each is independently a hydrogen atom or a lower alkyl group optionally having a substituent(s),
R⁵ is a lower alkyl group,
R⁶ and R⁷ are the same or different and each is independently a hydrogen atom or a lower alkyl group optionally having a substituent(s), or R⁶ and R⁷ form a C₃₋₈ cycloalkane ring together with a carbon atom bonded thereto, and
R⁸, R ⁹, R¹⁰ and R¹¹ are the same or different and each is independently a hydrogen atom or a halogen atom.

18. The compound according to claim 17, wherein, in the formula (8), R² and R³ are hydrogen atoms, and
R⁶ and R⁷ are the same or different and each is independently a methyl group, an ethyl group or a propyl group, or R⁶ and R⁷ form a cyclopropane ring, a cyclobutane ring or a cyclopentane ring together with a carbon atom bonded thereto, or a chemically acceptable salt thereof.

19. A compound represented by the following formula (15), or a chemically acceptable salt thereof:
